# EUROPEAN PATENT SPECIFICATION

(11) **EP 3 177 226 B1**
(45) Date of publication and mention of the grant of the patent: **26.09.2018**
(21) Application number: 15749927.8
(22) Date of filing: 31.07.2015
(51) Int. Cl.: A61B 18/14, A61M 25/00, A61L 29/06, A61L 29/10, A61M 25/01, A61L 29/04

(54) **THIN-FILM COATED FLUOROPOLYMER CATHETER**
DÜNNFILMBESCHICHTETER FLUORPOLYMERKATHETER
CATHÉTER DE POLYMÈRE FLUORÉ REVÊTU D'UN FILM MINCE

(30) Priority: 06.08.2014 US 201462033913 P
(43) Date of publication of application: 14.06.2017
(73) Proprietor: Cook Medical Technologies LLC, Bloomington, IN 47404 (US)
(72) Inventor: MARTINEZ, Michelle D., Winston-Salem, NC 27104 (US); SIGMON, John Crowder, Winston-Salem, NC 27101 (US); MCLAWHORN, Tyler Evans, Winston-Salem, NC 27106 (US); GITTARD, Shaun, Winston-Salem, NC 27101 (US); SURTI, Vihar, Winston-Salem, NC 27104 (US); HRNICEK, Jillian, Arvada, Colorado 80004 (US)
(74) Representative: Garratt, Peter Douglas
(86) International application number: PCT/US2015/043241
(87) International publication number: WO 2016/022430

(56) References cited:
- WO-A1-95/13704
- US-A- 4 557 957
- US-A- 5 554 176
- US-A1- 2004 230 188
- US-A1- 2008 051 634
- US-A1- 2013 274 736
- US-A1- 2014 188 109

## Description

### TECHNICAL FIELD

The present invention relates to fluoropolymer catheter-type medical devices having a thin-film ceramic or metallic coating that is applied by techniques such as Atomic Layer Deposition (ALD).

### BACKGROUND OF THE INVENTION

Fluoropolymers, such as PTFE, FEP, etc. are popular materials for making medical catheters, due to desirable properties including low friction coefficient, high dielectric coefficient, chemical resistance, and high melting temperature. However, there are also some challenges with using this class of material.

Fluoropolymers have an extremely low surface energy, which gives them their low friction properties. Unfortunately, this low surface energy makes it difficult to bond any materials to the catheter. Surface treatments, such as chemical etching and plasma etching have been used to improve the surface energy, however, these treatments have limited effectiveness and their effects are reversible.

One example of this challenge is in sphincterotomes. This type of device is used in endoscopy to treat biliary strictures and bile duct stones. These devices are generally made from PTFE due to the desirable properties listed above. Sphincterotomes have various inks applied on their distal end to aid in visualization, positioning, etc. Applying these inks to the sphincterotome is a challenging process and requires numerous treatment steps and specialized inks.

Thus, there exists a need for catheters and sphincterotomes having the desirable properties of a fluoropolymer material, but also allowing for portions of the device to have surface properties that allow better adhesion of ink coatings.

Reference is directed to US 2008/0051634 which discloses an outer sheath of a metal film provided on an outer peripheral surface of a tube base layer of a channel tube which is provided within an insertion section and has flexibility.

Reference is further directed to WO 95/13704 which discloses anti-microbial coatings and powders and method of forming same on medical devices. The coatings are preferably formed by depositing an anti-microbial biocompatible metal by vapour deposition techniques to produce atomic disorder in the coating such that a sustained release of metal ions sufficient to produce an anti-microbial effect is achieved.

### SUMMARY OF THE INVENTION

The invention is defined in appended independent claim 1, preferred embodiments are described in the dependent claims.

This invention provides a fluoropolymer medical device, such as a catheter or sphincterotome, having a thin-film coating in one or more places on the device. The thin-film coating is deposited using either a chemical or physical deposition technique. The thin-film coating may be either a ceramic or a metallic coating, where an outer coating is adhered to the ceramic coating. The outer coating may be either an ink coating or an adhesive coating. The ink coating may be a conducting ink or a radiopaque marker. In embodiments having a thin-film ceramic coating in more than one place on the fluoropolymer medical device (e.g., in two places), a first outer coating may be adhered to a first ceramic coating and a second outer coating may be adhered to a second ceramic coating, where the first and second outer coatings may be the same or different. Embodiments of the invention having a metallic coating or a conducting ink adhered to a ceramic coating may function as a bipolar device (e.g. bipolar sphincterotome).

In one aspect of the invention is provided a fluoropolymer medical device in the form of an elongated fluoropolymer body having an outer surface, a proximal end portion, a distal end portion, and a lumen extending at least partially through the distal end portion. A ceramic coating is deposited on the outer surface of the elongated tubular fluoropolymer body by chemical or physical deposition techniques. An outer coating is adhered to the ceramic coating. In some embodiments, the deposition technique is atomic layer deposition. In some embodiments, the outer coating is an ink coating, such as a conducting ink. In certain embodiments, the outer coating is an adhesive.

Also disclosed is a fluoropolymer medical device in the form of an elongated fluoropolymer body having an outer surface, a proximal end portion, a distal end portion, and a lumen extending at least partially through the distal end portion. A metal or metallic coating is deposited on the outer surface of the elongated tubular fluoropolymer body by chemical or physical deposition techniques. In some embodiments, the deposition technique is atomic layer deposition.

### BRIEF DESCRIPTION OF THE DRAWINGS

FIG. 1 Drawing of a bipolar sphincterotome with ceramic coating and ink coating
FIG. 2A Cross-sectional view of sphincterotome showing lumens with ceramic coating and ink coating extending partially around the circumference
FIG. 2B Cross sectional view of sphincterotome showing a single metallic coating.
FIG. 2C Cross sectional view of sphincterotome showing ceramic coating and ink coating extending around entire circumference.
FIG. 2D Cross sectional view showing simple catheter with ceramic coating and outer coating.
FIG. 3 Drawing of bipolar sphincterotome with a single metallic coating
FIG. 4. Drawing of introducer catheter showing ink coating on distal end and adhesive coating on proximal end.

### DETAILED DESCRIPTION

The embodiments are described with reference to the drawings in which like elements are referred to by like numerals. The relationship and functioning of the various elements of the embodiments are better understood by the following detailed description. However, the embodiments as described below are by way of example only, and the invention is not limited to the embodiments illustrated in the drawings. For example, although the figures and description below generally are in terms of a sphincterotome, the present invention broadly encompasses any type of fluoropolymer catheter having the thin-film coatings disclosed herein. It should also be understood that the drawings are not to scale and in certain instances details have been omitted, which are not necessary for an understanding of the embodiments, such as conventional details of fabrication and assembly.

The fluoropolymer medical devices of the invention are coated with a thin-film coating. In some embodiments, the thin-film coating is a ceramic coating. In other configurations not according to the invention, the thin-film coating is a metal coating. The thin-film coatings are deposited on the fluoropolymer medical device using either a chemical or a physical deposition technique. Chemical deposition includes, for example, Atomic Layer Deposition (ALD). Physical deposition includes, for example, Pulsed Laser Deposition (PLD), molecular beam epitaxy, sputter deposition, Laser Induced Forward Transfer (LIFT), Matrix Assisted Pulsed Laser Evaporation (MAPLE), and Matrix Assisted Pulsed Laser Evaporation Direct Write (MAPLEDW). Physical deposition techniques physically embed the coated material into the surface of the substrate material. Masking may be used to protect portions of the device from receiving the coating. In general, thin-film coatings of the invention have a thickness greater than or equal to about 10 nm. In certain embodiments, the thin-film coating is about 10 nm to about 25 nm.

ALD is a chemical vapor deposition process that provides a uniform coating. ALD uses reaction gases to form a thin layer of deposited material. In ALD, reactant gas pulses are separately introduced to the substrates to be coated. Growth is achieved through self-terminating surface reactions. Self-terminating means that only one monolayer of reactant gas species can be adsorbed to the surface during a pulse. The pulses containing reactant gases are separated by purging pulses where the reactor is flushed with an inert gas. The purging pulses ensure that the reactant gas pulses do not mix. By-products like excess reactants are also flushed away by the purging pulses. A complete set of reactant gas pulses and purging pulses needed to deposit a certain compound are referred to as a cycle. The reaction gases are introduced in pulses into a reactor containing the object to be coated. For example, Al₂O₃ may be deposited by introducing a pulse of trimethylaluminum, followed by a purging pulse of inert gas (e.g., nitrogen), followed by a pulse of water, and another purging pulse of nitrogen to remove excess water. Similarly, TiO₂ may be deposited by introducing a pulse of TiCl₄, followed by a purging pulse of nitrogen, followed by a pulse of water that reacts with the deposited TiCl₄ to form TiO₂, followed by another purging pulse to remove excess water and any by-products. The foregoing sequences of pulses may be repeated to form a coating of the desired thickness.

In certain embodiments the thin-film coating is a ceramic coating. Suitable ceramic coatings include Al₂O₃, CaO, CuO, Er₂O₃, Ga₂O₃, HfO₂, La₂O₃, MgO, Nb₂O₅, Sc₂O₃, SiO₂, Ta₂O₅, TiO₂, V_{X}O_{Y} (i.e., vanadium oxides), Y₂O₃, Yb₂O₃, ZnO, ZrO, AlN, GaN, TaC, TiC, WC, and hydroxyapatite. In certain embodiments, the ceramic coating is selected from one or more of Al₂O₃, TiO₂, SiO₂, and ZrO. Preferably, the ceramic coating comprises, or consists essentially of, Al₂O₃ (i.e., alumina).

An ink coating may be adhered to the ceramic coating described herein. The ink coating may be either a conductive ink or a radiopaque marker for use in visualization. Use of a conductive ink allows the device to function as a bipolar device. The use of an underlying ceramic coating, such as alumina, overcomes the problem with existing techniques for applying ink to fluoropolymer catheters through the improved adherence of an ink coating to the ceramic coating deposited on the surface of the fluoropolymer device.

In certain configurations not according to the invention, the thin-film coating is a metal/metallic coating. Suitable metals include silver, gold, copper, iridium, palladium, platinum and ruthenium. In certain configurations, the metal coating is selected from one or more of the foregoing metals. In cases where the thin-film coating is metallic, the ceramic coating may be omitted.

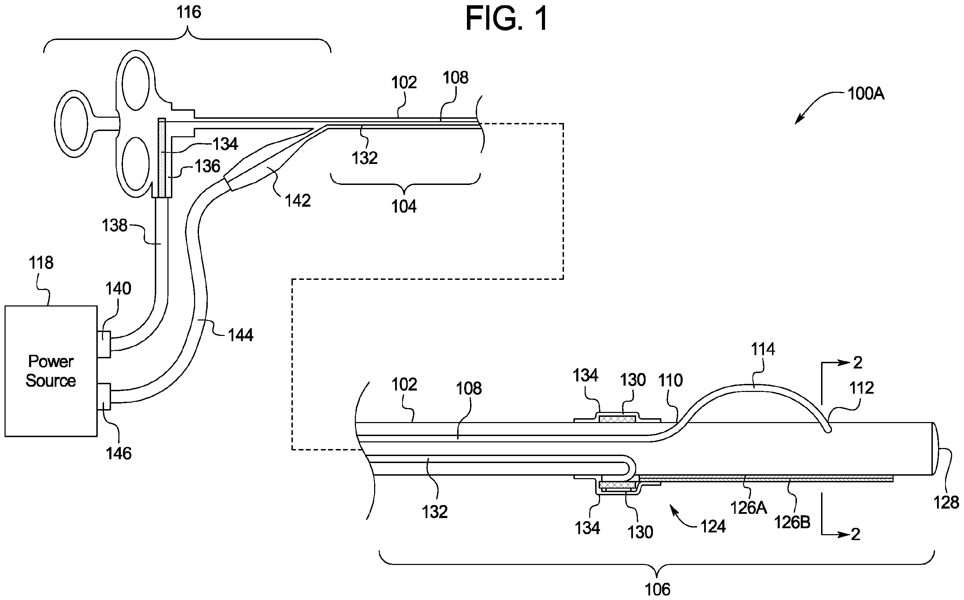

An exemplary embodiment of a thin-film coated fluoropolymer device of the invention is shown in FIG. 1. FIG. 1 shows a partially cross-sectional side view of an example bipolar sphincterotome 100A. The example bipolar sphincterotome 100A includes an elongated tubular fluoropolymer body 102 that has a proximal end portion 104 extending to a distal end portion 106. An electrically conductive cutting element 114 (e.g. a cutting wire) used to cut the sphincter muscle is located along the distal end portion 106. The cutting element 114 is connected to an electrical conductor 108 extending within a second lumen (not shown in FIG. 1) through at least a portion of the tubular body 102. In the exemplary embodiment of FIG. 1, the electrical conductor 108 extends from the proximal end portion 104 to the distal end portion 106. At the distal end portion 106, the cutting wire 114 protrudes from within the tubular body 102, through a first opening 110 of the tubular body 102, to outside the tubular body 102. Outside the tubular body 102, the cutting wire 114 may longitudinally extend substantially parallel with the tubular body 102 to a second opening or anchor point 112 of the tubular body 102 that is distal the first opening 110, where a distal end of the cutting wire 114 may re-enter and/or be fixedly attached to the tubular body 102.

The bipolar sphincterotome 100A may further include a return path 124. For the bipolar configuration, the return path 124 includes an outer coating 126B in the form of a conductive ink coating adhered to a ceramic coating 126A disposed on the outer surface of the tubular body 102 at the distal end portion 106. A return wire 132 is electrically coupled to the conductive ink coating 126B. The ceramic coating may have a thickness of greater than or equal to about 10 nm. In some embodiments, the ceramic coating is about 10 nm to about 25 nm in thickness. Additionally, the conductive ink 126B may have a viscosity of about 250 centipoise (cP), although other viscosities may be used, including up to about 10,000 cP. Also, a resistance of the conductive ink portion 126B may be in a range of about zero (or substantially zero) to ten Ohms, when measured longitudinally. An example conductive ink, which may or may not include all of the above described properties, may be AG-510 Silver Filled Electrically Conductive Screen Printable Ink/Coating by Conductive Compounds, Inc.

The ceramic coating 126A and the outer conductive ink coating 126B may extend distally past the anchor point 112. Extending the conductive ink coating 126B distally past the anchor point 112 may ensure or increase the likelihood that the return path 124 contacts the sphincter muscle (or tissue near the sphincter muscle) to make a proper connection at the treatment site. Additionally, the conductive ink coating 126B may distally extend to a position before a distal tip 128 or sufficiently away from an opening of a wire guide lumen (not shown in FIG. 1) at the distal tip 128 so that a wire guide in the wire guide lumen is not part of or is isolated from the return path 124. In addition, the ceramic coating 126A and conductive ink coating 126B may proximally extend past the first opening 110.

The conductive ink coating 126B may be electrically coupled to a return wire 132, which may form and/or be part of the return path 124. The return wire 132 may extend within the tubular member 102 from where the return wire 132 is electrically coupled to the conductive ink coating 126B to the proximal portion 104. The return wire 132 may extend within the tubular member 102 generally or substantially parallel to the electrical conductor 108. In addition, the return wire 132 may extend within the tubular member 102 in various locations relative to the electrical conductor 108. FIG. 1 shows the electrical conductor 108 and the return wire 132 generally in the same cross-sectional plane. However, the return wire 132 may be disposed within the tubular body 102 in various locations relative to the electrical conductor 108. Also, the return wire 132 may be disposed and/or extend within a lumen of the tubular body 102, or alternatively, may be embedded within and/or coextruded with the tubular body 102.

A conductive ink coating 126B may be electrically coupled to the return wire 132 in various ways. For example, as shown in FIG. 1, the conductive ink coating 126B may proximally extend to a conductive ring or cannula 130, which may electrically couple the conductive ink coating 126B to the return wire 132. In some example embodiments, the conductive cannula 130 may be attached or crimped to the outer surface of the tubular body 102. The conductive cannula 130 may be made of metal, such as stainless steel, silver, gold, tantalum, or tungsten, as examples. The conductive ink coating 126B is in contact with at least a portion of the conductive cannula 130 so that the conductive ink coating 126B and the conductive cannula 130 are electrically coupled, and the conductive cannula 130 is part of the return path 124. As shown in FIG. 1, the return wire 132 may be connected to the conductive cannula 130 to be electrically coupled with the conductive ink coating 126B. For example, the return wire 132 may be curled at its distal end to extend to the outer surface of the tubular member 102, and the conductive cannula may be crimped to the tubular body 102 over the distal end of the return wire 132.

In some embodiments, the bipolar sphincterotome 100A may further include a tube 134 disposed over the conductive cannula 130 and the conductive ink coating 126B. As shown in FIG. 1, the tube 134 may distally extend to the first opening 110 in the tubular body 102, or alternatively to a position in between the cannula 130 and the first opening 110. In some embodiments, the tube 134 may be a shrink tube 134 that conforms to the surface that the shrink tube 134 is covering, such as when heat is applied to the shrink tube 134. The tube 134 may have a thickness of about 0,00508 mm (0.0002 inches) although other thicknesses may be used. The tube 134 may be disposed over the cannula 130 to provide a relief to the strain caused by varying flexibilities between the tubular body 102 (which may be relatively flexible) and the metal cannula 130 (which may be relatively rigid). Additionally, the tube 134 may provide a protective coating or scratch resistance, which may prevent or minimize the conductive ink coating 126B from being scratched off.

For some example embodiments of the tube 134, an inner surface of the tube 134 may be coated with one or more conductive materials, such as a conductive ink, a conductive powder, a conductive adhesive, or combinations thereof, as examples. The conductive material may be the same material as or may be a different material then the conductive ink coating 126B. The tube 134, with an inner surface coated with a conductive material, may enhance electrical continuity between the conductive ink coating 126B and the conductive cannula 130. Other arrangements for coupling the conductive ink coating 126B with the return wire 132 are described in U.S. Patent Publication No. US2014/0188109.

The bipolar sphincterotome 100A may further include a handle assembly 116 coupled to the proximal portion 104 and/or a proximal end of the electrical conductor 108. The handle assembly 116 may be operatively coupled to the electrical conductor 108 to move the cutting wire 114 between a relaxed state and a cutting state. For example, the handle assembly 116 may be configured to move the cutting wire 114 from the relaxed state to the cutting state by proximally pulling the cutting wire 108 taut. When the electrical conductor 108 is pulled, the distal portion 106 of the tubular member 102 may bow or curl, forming an arc. The taut cutting wire 114 may form a secant of the arc. When the distal portion 106 is curled and the cutting wire 114 is taut, the distal portion 106 and the cutting wire 114 may be configured or in position to cut the sphincter muscle. The handle assembly 116 may also be configured to release or distally push the electrical conductor 108 to uncurl the distal portion 106 and to move the cutting wire 114 from the taut state to the relaxed state. When the distal portion 106 is uncurled (or at least in a position that is curled to a lesser degree than when the cutting wire 114 is taut) and the cutting wire 114 is in the relaxed state, the distal portion 106 and the cutting wire 114 may not be configured to cut the sphincter muscle and/or may be configured or in position to be moved to and from the treatment site. Alternatively, the sphincterotome may have a precurved distal tip as described in U.S. Patent Publication No. 2010/0057077.

Both the electrical conductor 108 and the return wire 132 may be electrically coupled to a power source 118, such as a radio frequency (RF) generator or an electrosurgical unit (ESU) that supplies electrical current to the electrical conductor 108 to perform the electrosurgery. In one example embodiment, the electrical conductor 108 may be electrically coupled to the power source 118 by proximally extending to the handle assembly 116, where the proximal end of the electrical conductor 108 may be connected to a metallic pin 134 that extends to a port 136 of the handle assembly 116. The metallic pin 134 and/or the port 136 may be adaptable to connect to supply cabling 138 that may connect to an active port 140 of the power source 118.

The return wire 132 may be electrically coupled to the power source 118 by distally extending through a side port 142 connected to the tubular body 102, where a proximal end of the return wire 132 may be connected to return cabling 144, such as by soldering the return wire with one or more wires of the return cabling 144. Alternatively, the return wire 132 may be connected to the return cabling 144 by crimping the return cabling to the return wire 132 disposed inside a metal cannula. The return cabling 144 may be adaptable to connect to a return port 146 of the power source 118. When the power source 118 is activated, the power source 118 may deliver electric current to the electrical conductor 108 via the supply cabling 138 and the metallic pin 134. The electrical current may pass through the electrical conductor 108 to the cutting wire 114, where electrosurgery may be performed on a sphincter muscle. The electrical current may pass through the sphincter muscle, which acts as a load, and then along the return path 124, including the conductive ink coating 126B and the return path, back to the power source 118 via the return cabling 144.

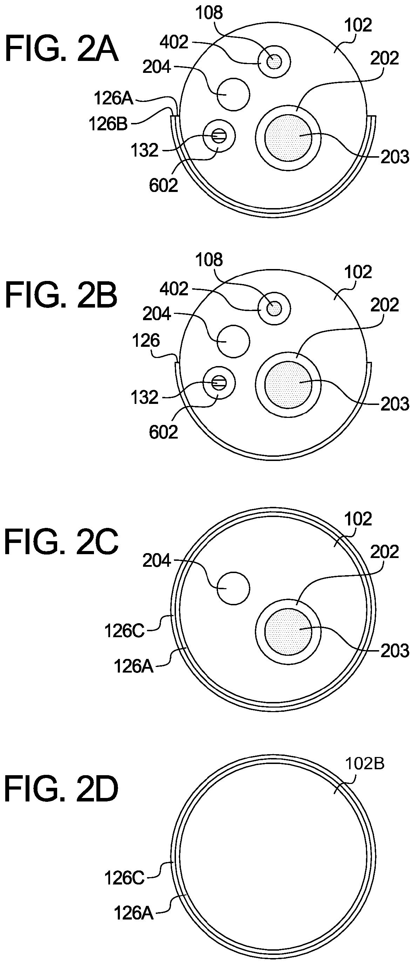

In certain embodiments, a wire guide lumen extends through at least a portion of the distal end portion. In general, the tubular body 102 may have a single lumen, or multiple lumens, i.e., two or more lumens. FIG. 2A shows a cross-sectional view of the tubular body along the line 2-2. In the embodiment in FIG. 2A, the tubular body has four lumens. Lumen 202 is adapted to receive a wire guide 203 that may be movably disposed within the wire guide lumen 202. The lumen 204 may be configured for the passage of fluids or contrast therethrough. Lumen 402 is adapted to receive an electrical conductor wire 108. Although the lumens in FIG. 2A are shown with circular cross-sectional shapes, other lumen shapes are possible. As further shown in FIG. 2A, a ceramic coating 126A and conducting ink coating 126B may be circumferentially disposed partially around the outer surface of the tubular body 102. As shown in FIG. 2C, a ceramic coating 126A may extend around the entire circumference of the tubular body 102 with an outer coating 126C that is a radiopaque ink adhered thereto.

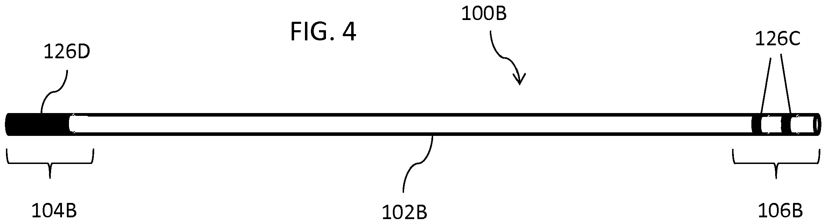

In other embodiments of the invention, the fluoropolymer medical device may be a catheter or sphincterotome where the distal end portion has one or more ceramic coatings and one or more outer coatings adhered to the ceramic coatings, where the outer coatings are ink coatings serving as a radiopaque markers. In FIG. 4 is shown a simple catheter having two outer coatings 126C adhered to two inner coatings (not shown) at the distal end portion 106B of tubular body 102B. In certain embodiments, the fluoropolymer medical device may be an introducer catheter or guiding sheath (e.g., FIG. 4) that may be used to introduce a stent, a balloon, or other diagnostic or interventional device. FIG. 2D shows a representative cross-sectional view of such a tubular body 102B where a ceramic coating 126A and an outer coating 126C (e.g., an ink coating) extend around the circumference of tubular body 102B.

In other embodiments, the outer coating may be an adhesive coating. For example, in FIG. 4, an outer coating 126D may be located at the proximal end portion 104B of a catheter, where the outer coating 126D is an adhesive coating (underlying ceramic coating not shown) used to better adhere a handle to the proximal end portion of a catheter. Although a catheter is generally represented in FIG. 4, an outer coating that is an adhesive coating may also be used on the proximal end portion of a sphincterotome, such as those described elsewhere herein. Further as indicated in FIG. 4, the invention provides fluoropolymer medical devices having multiple ceramic coatings and outer coatings where the outer coatings may be the same or different. An inner ceramic coating and an outer adhesive coating may also be used without coatings on the distal end portion. Where multiple outer coatings are ink coatings, the ink coatings may be the same or different materials, depending on the particular application.

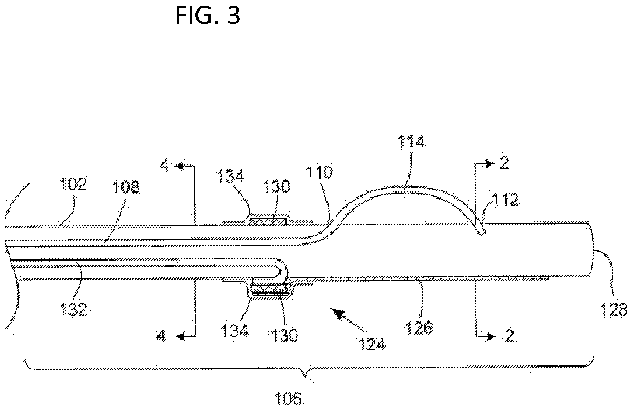

In yet other embodiments of the invention, the thin-film coating may be a metal or metallic coating. Shown in FIG. 3 is an exemplary embodiment of the distal end portion of a bipolar sphincterotome 100 where the thin-film coating is a metal coating 126. The metal coating 126 is electrically coupled to the return wire in the same fashion as described above for the embodiment of FIG. 1. In the embodiment of FIG. 3, however, the ceramic and conductive ink coatings may be omitted since the thin-film coating itself is metallic and therefore conducting on its own. In FIG. 2B is shown a cross-sectional view of an embodiment where the thin-film coating is a metal coating 126 extending partially around the circumference of the tubular body 102.

The above figures and disclosure are intended to be illustrative and not exhaustive. This description will suggest many variations and alternatives to one of ordinary skill in the art. All such variations and alternatives are intended to be encompassed within the scope of the attached claims. Those familiar with the art may recognize other equivalents to the specific embodiments described herein which equivalents are also intended to be encompassed by the attached claims.

## Claims

1. A fluoropolymer medical device comprising:
an elongated tubular fluoropolymer body (102) having an outer surface, a proximal end portion, a distal end portion, and a lumen extending at least partially through the distal end portion;
one or more ceramic coatings (126A), the one or more ceramic coatings being deposited on the outer surface of the elongated tubular fluoropolymer body by a technique selected from atomic layer deposition, pulsed laser deposition, molecular beam epitaxy, sputter deposition, laser induced forward transfer, matrix assisted pulsed laser evaporation, and matrix assisted pulsed laser evaporation direct write; and
one or more outer coatings (126B), the one or more outer coatings independently comprising an ink coating or an adhesive coating, the one or more outer coatings being adhered to the one or more ceramic coatings.

2. The fluoropolymer medical device of claim 1, wherein the medical device is a sphincterotome.

3. The fluoropolymer medical device of claim 2, wherein the sphincterotome is a bipolar sphincterotome (100A).

4. The fluoropolymer medical device of claim 2 or 3 comprising a first ceramic coating deposited on the distal end portion.

5. The fluoropolymer medical device of claim 4, wherein the one or more outer coatings comprises the ink coating (126B) and the ink coating is adhered to the first ceramic coating (126A).

6. The fluoropolymer medical device of claim 5, wherein the ink coating is a conductive ink.

7. The fluoropolymer medical device of claim 1, wherein the medical device is an introducer catheter (100B).

8. The fluoropolymer medical device of any of claims 1-5 or 7, wherein the one or more outer coatings comprises the ink coating and the ink coating is a radiopaque marker.

9. The fluoropolymer medical device of any of claims 1-8, wherein the one or more ceramic coatings or the first ceramic coating are each independently selected from one or more of Al₂O₃, TiO₂, ZrO, and SiO₂.

10. The fluoropolymer medical device of claim 9, wherein the one or more ceramic coatings or the first ceramic coating comprises Al₂O₃.

11. The fluoropolymer medical device of any of claims 3 and 4-6 when depending on 3, wherein the bipolar sphincterotome further comprises:
an electrically conductive cutting element (114) located along the distal end portion, the cutting element being connected to an electrical conductor (108) extending within a second lumen extending through at least a portion of the tubular body, the cutting element extending exteriorly of the tubular body.

12. The fluoropolymer medical device of claim 6, wherein the bipolar sphincterotome further comprises:
an electrically conductive cutting element (114) located along the distal end portion, the cutting element being connected to an electrical conductor (108) extending within a second lumen extending through at least a portion of the tubular body, the cutting element extending exteriorly of the tubular body; and
a return wire electrically coupled to the conducting ink, the return wire being disposed within the elongated fluoropolymer tubular body.

13. The fluoropolymer medical device of any of the preceding claims comprising a second ceramic coating deposited on the proximal end portion.

14. The fluoropolymer medical device of claim 13 comprising an adhesive coating adhered to the second ceramic coating.

15. The fluoropolymer medical device of any of the preceding claims wherein the one or more ceramic coatings, the first ceramic coating, or the second ceramic coating, each has a thickness of greater than or equal to about 10 nm .

16. The fluoropolymer medical device of claim 15 wherein the thickness is about 10 nm to about 25 nm.

17. The fluoropolymer medical device of any of the preceding claims comprising:
a plurality of ceramic coatings deposited on the distal end portion; and
a plurality of ink coatings adhered to each of the ceramic coatings of the plurality of ceramic coatings.

## Patentansprüche

1. Medizinische Fluorpolymervorrichtung, umfassend: einen langgestreckten rohrförmigen Fluorpolymerkörper (102) mit einer Außenoberfläche, einem proximalen Endteil, einem distalen Endteil und einem Lumen, das wenigstens teilweise durch den distalen Endteil verläuft;
eine oder mehrere keramische Beschichtungen (126A), wobei die eine oder mehreren keramischen Beschichtungen durch ein Verfahren ausgewählt aus Atomschichtabscheidung, gepulster Laserabscheidung, Molekülstrahlepitaxie, Sputterabscheidung, laserinduziertem Vorwärtstransfer, matrixunterstützter gepulster Laserverdampfung und matrixunterstütztem gepulstem Laserverdampfungs-Direktschreiben auf der Außenoberfläche des langgestreckten rohrförmigen Fluorpolymerkörpers aufgebracht sind; und
eine oder mehrere Außenbeschichtungen (126B), wobei die eine oder mehreren Außenbeschichtungen unabhängig eine Tintenbeschichtung oder eine Haftbeschichtung umfassen, wobei die eine oder mehreren Außenbeschichtungen an der einen oder den mehreren keramischen Beschichtungen haften.

2. Medizinische Fluorpolymervorrichtung gemäß Anspruch 1, wobei die medizinische Vorrichtung ein Sphinkterotom ist.

3. Medizinische Fluorpolymervorrichtung gemäß Anspruch 2, wobei das Sphinkterotom ein bipolares Sphinkterotom (100A) ist.

4. Medizinische Fluorpolymervorrichtung gemäß Anspruch 2 oder 3, umfassend eine erste keramische Beschichtung, die auf dem distalen Endteil aufgebracht ist.

5. Medizinische Fluorpolymervorrichtung gemäß Anspruch 4, wobei die eine oder mehreren Außenbeschichtungen die Tintenbeschichtung (126B) umfassen und die Tintenbeschichtung an der ersten keramischen Beschichtung (126A) haftet.

6. Medizinische Fluorpolymervorrichtung gemäß Anspruch 5, wobei die Tintenbeschichtung eine leitfähige Tinte ist.

7. Medizinische Fluorpolymervorrichtung gemäß Anspruch 1, wobei die medizinische Vorrichtung ein Einführkatheter (100B) ist.

8. Medizinische Fluorpolymervorrichtung gemäß einem der Ansprüche 1-5 oder 7, wobei die eine oder mehreren Außenbeschichtungen die Tintenbeschichtung umfassen und die Tintenbeschichtung eine röntgendichte Markierung ist.

9. Medizinische Fluorpolymervorrichtung gemäß einem der Ansprüche 1-8, wobei die eine oder mehreren keramischen Beschichtungen oder die erste keramische Beschichtung jeweils unabhängig ausgewählt ist aus einem oder mehreren von Al₂O₃, TiO₂, ZrO und SiO₂.

10. Medizinische Fluorpolymervorrichtung gemäß Anspruch 9, wobei die eine oder mehreren keramischen Beschichtungen oder die erste keramische Beschichtung Al₂O₃ umfasst.

11. Medizinische Fluorpolymervorrichtung gemäß einem der Ansprüche 3 und 4-6, wenn abhängig von 3, wobei das bipolare Sphinkterotom ferner umfasst:
ein elektrisch leitfähiges Schneidelement (114), das entlang des distalen Endteils angeordnet ist, wobei das Schneidelement mit einem elektrischen Leiter (108) verbunden ist, der innerhalb eines zweiten Lumens verläuft, das durch wenigstens einen Teil des rohrförmigen Körpers verläuft, wobei das Schneidelement außerhalb des rohrförmigen Körpers verläuft.

12. Medizinische Fluorpolymervorrichtung gemäß Anspruch 6, wobei das bipolare Sphinkterotom ferner umfasst:
ein elektrisch leitfähiges Schneidelement (114), das entlang des distalen Endteils angeordnet ist, wobei das Schneidelement mit einem elektrischen Leiter (108) verbunden ist, der innerhalb eines zweiten Lumens verläuft, das durch wenigstens einen Teil des rohrförmigen Körpers verläuft, wobei das Schneidelement außerhalb des rohrförmigen Körpers verläuft; und
einen Rückführdraht, der elektrisch an die leitfähige Tinte gekoppelt ist, wobei der Rückführdraht innerhalb des langgestreckten rohrförmigen Fluorpolymerkörpers angeordnet ist.

13. Medizinische Fluorpolymervorrichtung gemäß einem der vorstehenden Ansprüche, umfassend eine zweite keramische Beschichtung, die auf dem proximalen Endteil aufgebracht ist.

14. Medizinische Fluorpolymervorrichtung gemäß Anspruch 13, umfassend eine Haftbeschichtung, die an der zweiten keramischen Beschichtung haftet.

15. Medizinische Fluorpolymervorrichtung gemäß einem der vorstehenden Ansprüche, wobei die eine oder mehreren keramischen Beschichtungen, die erste keramische Beschichtung oder die zweite keramische Beschichtung jeweils eine Dicke von mehr als oder etwa gleich 10 nm aufweisen.

16. Medizinische Fluorpolymervorrichtung gemäß Anspruch 15, wobei die Dicke etwa 10 nm bis etwa 25 nm beträgt.

17. Medizinische Fluorpolymervorrichtung gemäß einem der vorstehenden Ansprüche, umfassend:
eine Vielzahl von keramischen Beschichtungen, die auf dem distalen Endabschnitt aufgebracht sind; und
eine Vielzahl von Tintenbeschichtungen, die an jeder der keramischen Beschichtungen aus der Vielzahl von keramischen Beschichtungen haftet.

## Revendications

1. Dispositif médical à fluoropolymère comprenant :
un corps de fluoropolymère tubulaire allongé (102) ayant une surface externe, une partie terminale proximale, une partie terminale distale et un lumen s'étendant, au moins partiellement, le long de la partie terminale distale ;
un ou plusieurs revêtement(s) céramique(s) (126A), l'un ou les plusieurs revêtement(s) céramique(s) étant déposé(s) sur la surface externe du corps de fluoropolymère tubulaire allongé par une technique choisie parmi un dépôt par couche atomique, un dépôt par laser pulsé, une épitaxie par faisceau moléculaire, un dépôt par pulvérisation cathodique, un transfert avant induit par laser, une évaporation par laser pulsé assistée par matrice et une écriture directe via une évaporation par laser pulsé assistée par matrice ;
et
un ou plusieurs revêtement(s) externe(s) (126B), l'un ou les plusieurs revêtement(s) externe(s) comprenant indépendamment un revêtement d'encre ou un revêtement adhésif, l'un ou les plusieurs revêtement(s) externe(s) étant fixé(s) à l'un ou aux plusieurs revêtement(s) céramique(s).

2. Dispositif médical à fluoropolymère de la revendication 1, le dispositif médical étant un sphinctérotome.

3. Dispositif médical à fluoropolymère de la revendication 2, dans lequel le sphinctérotome est un sphinctérotome bipolaire (100A).

4. Dispositif médical à fluoropolymère des revendications 2 ou 3 comprenant un premier revêtement céramique déposé sur la partie terminale distale.

5. Dispositif médical à fluoropolymère de la revendication 4, dans lequel l'un ou les plusieurs revêtement(s) externe(s) comprend/comprennent le revêtement d'encre (126B)
et le revêtement d'encre est fixé au premier revêtement céramique (126A).

6. Dispositif médical à fluoropolymère de la revendication 5, dans lequel le revêtement d'encre est une encre conductrice.

7. Dispositif médical à fluoropolymère de la revendication 1, le dispositif médical étant un cathéter d'introduction (100B).

8. Dispositif médical à fluoropolymère de l'une quelconque des revendications 1 à 5 ou 7, dans lequel l'un ou les plusieurs revêtement(s) externe(s) comprend/comprennent le revêtement d'encre, et le revêtement d'encre est un marqueur radio-opaque.

9. Dispositif médical à fluoropolymère de l'une quelconque des revendications 1 à 8, dans lequel l'un ou les plusieurs revêtement(s) céramique(s) ou bien le premier revêtement céramique est/sont chacun indépendamment choisi(s) entre un ou plusieurs élément (s) parmi les Al₂O₃, TiO₂, ZrO et SiO₂.

10. Dispositif médical à fluoropolymère de la revendication 9, dans lequel l'un ou les plusieurs revêtement(s) céramique(s) ou bien le premier revêtement céramique comprend/comprennent de l'Al₂O₃.

11. Dispositif médical à fluoropolymère de l'une quelconque des revendications 3 et 4 à 6 quand elles dépendent de la 3, dans lequel le sphinctérotome bipolaire comprend en outre :
un élément coupant conducteur d'un point de vue électrique (114) situé le long de la partie terminale distale, l'élément coupant étant relié à un conducteur électrique (108) s'étendant dans un deuxième lumen qui s'étend à travers au moins une partie du corps tubulaire, l'élément coupant s'étendant à l'extérieur du corps tubulaire.

12. Dispositif médical à fluoropolymère de la revendication 6, dans lequel le sphinctérotome bipolaire comprend en outre :
un élément coupant conducteur d'un point de vue électrique (114) situé le long de la partie terminale distale, l'élément coupant étant relié à un conducteur électrique (108) s'étendant dans un deuxième lumen qui s'étend à travers au moins une partie du corps tubulaire, l'élément coupant s'étendant à l'extérieur du corps tubulaire ; et
un fil de retour couplé électriquement avec l'encre conductrice, le fil de retour étant disposé au sein du corps tubulaire à fluoropolymère allongé.

13. Dispositif médical à fluoropolymère de l'une quelconque des revendications précédentes comprenant un deuxième revêtement céramique déposé sur la partie terminale proximale.

14. Dispositif médical à fluoropolymère de la revendication 13 comprenant un revêtement adhésif fixé au deuxième revêtement céramique.

15. Dispositif médical à fluoropolymère de l'une quelconque des revendications précédentes, dans lequel l'un ou les plusieurs revêtement(s) céramique(s), le premier revêtement céramique ou le deuxième revêtement céramique, a/ont chacun une épaisseur supérieure ou égale à 10 nm environ.

16. Dispositif médical à fluoropolymère de la revendication 15, dans lequel l'épaisseur est de 10 nm environ jusqu'à 25 nm environ.

17. Dispositif médical à fluoropolymère de l'une quelconque des revendications précédentes comprenant :
une pluralité de revêtements céramiques déposés sur la partie terminale distale ; et
une pluralité de revêtements d'encre fixés à chacun des revêtements céramiques de la pluralité de revêtements céramiques.
